# EUROPEAN PATENT APPLICATION

(11) **EP 4 674 394 A1**
(43) Date of publication of application: **07.01.2026**
(21) Application number: 24186009.7
(22) Date of filing: 02.07.2024
(51) Int. Cl.: A61F 13/00, A61F 13/0203, A61F 13/02, G06Q 30/016

(54) **A MEDICAL DRESSING COMPRISING A MACHINE-READABLE CODE**

(71) Applicant: Mölnlycke Health Care AB, 431 21 Mölndal (SE)
(72) Inventor: FLACH, Niclas, 441 35 ALINGSÅS (SE); MELIN, Daniel, 435 30 MÖLNLYCKE (SE); JAKOBSSON, Conny, 443 38 LERUM (SE); NIELSEN, Kent, 3650 ØLSTYKKE (DK)
(74) Representative: Kransell & Wennborg KB

(57) **Abstract**

The present disclosure generally relates to a medical dressing (1) comprising at least a backing layer (2) and an adhesive skin-contact layer (3); the backing layer (2) being transparent or translucent and having a top side and a bottom side, wherein the medical dressing comprises a machine-readable code (4) arranged between the top side of the backing layer (2) and the adhesive skin-contact layer (3), wherein the medical dressing (1) further comprises a reinforcement element (5) in direct contact with and below the machine-readable code (4).

## Description

### TECHNICAL FIELD

The present disclosure generally relates to a medical dressing comprising at least a backing layer and an adhesive skin-contact layer; the backing layer being transparent or translucent and having a top side and a bottom side, wherein the medical dressing comprises a machine-readable code arranged between the top side of the backing layer and the adhesive skin-contact layer.

### BACKGROUND

Adhesive medical dressings are frequently used in wound care, both for the purpose of treating wounds and scars and for the purpose of preventing these from occurring in the first place.

An adhesive medical dressing typically comprises an adhesive skin-contact layer arranged to contact the skin or wound of a patient, as well as a top layer, often referred to as a backing layer. In many cases, particularly when the dressing is to be applied to moderate or highly exuding wounds, the dressing further comprises a wound pad arranged between the backing layer and the adhesive skin-contact layer. To protect the dressing from contamination, a release liner is applied to the adhesive skin-contact layer.

Adhesive medical dressings are typically individually packed in pouches, which keep the dressings sterile prior to use. A plurality of pouches housing the dressings are generally contained and stored in an outer package (a "multi-pack").

The pouch and the outer package may contain a variety of product-identifying information. For example, lot lumbers, batch numbers, and dressing-specific information may be printed on the pouch. Furthermore, instructions relating to a correct use or application of the dressing may be provided in an instruction manual ("Instructions for use", IFU), which comes with the package. Such information may also be printed on the outer package.

However, in many cases, usage- or product-related information (as provided in e.g. an IFU or on the outer package) is not present in the actual care situation and may easily become lost or dislocated. Also, once the dressing has been applied to the wound or skin of a patient, the pouch is typically discarded, and the product- or usage-related information is thereby lost.

In view of this, it is desirable to print product- or usage- related markings on the actual product, i.e. wound dressing, such that the markings are visibly observable even when the medical dressing has been applied to the skin or wound of a patient.

Product-identifying information as well as usage-specific information may be encoded in machine-readable codes.

US 2011/183712A1 relates to a wound dressing comprising a QR code permanently attached to the outer side of a carrier layer, i.e. top layer of the dressing. The QR code provides a uniform resource locator of an internet site containing usage-specific and/or technical data relating to the wound dressing.

A problem associated with printing machine-readable codes, e.g. QR codes on an outer surface of the dressing is that the machine-readable code may become damaged during use, i.e. due to wear and tear. Printing on the outermost surface of the dressing, i.e. top layer, enhances the risk of the printed marking becoming chafed during use. In this regard, the machine-readable code may become difficult to scan or read by an electronic device, such as a mobile phone.

Furthermore, the top layer, i.e. backing layer, of the dressing is typically a thin (and flimsy) film, which may render the dressing prone to wrinkle formation, particularly at the edges of the dressing. After prolonged use of the dressing, the edges of the dressing may start to "curl". If a machine-readable code is arranged in an edge portion of the dressing, the problems associated with proper scanning of the machine-readable code may be further enhanced by such edge curling.

Moreover, upon absorption of wound exudate, the wound pad typically swells, which may form irregularities in the overlying thin backing film. Such irregularities may render a printed machine-readable code arranged on a backing film above the wound pad difficult to read or scan.

In view of the scenarios described above, it would be desirable to provide a medical dressing comprising a printed machine-readable code, wherein the protection of the machine-readable code is improved such that the machine-readable code is scannable and readable throughout the use of the dressing.

### SUMMARY

In view of the above-mentioned problems, it is an object of the present disclosure to provide improvements in the field of medical dressings comprising printed machine-readable codes.

According to a first aspect, there is provided a medical dressing comprising at least a backing layer and an adhesive skin-contact layer; the backing layer being transparent or translucent and having a top side and a bottom side, wherein the medical dressing comprises a machine-readable code arranged between the top side of the backing layer and the adhesive skin-contact layer, wherein the medical dressing further comprises a reinforcement element in direct contact with and below the machine-readable code.

The present disclosure is based on the realization that the reinforcement element renders the area underlying the machine-readable code more rigid. This may enhance the protection of the machine-readable code as well as improve the readability of the machine-readable code by an electronic device, e.g. a mobile phone.

For example, if the machine-readable code is arranged in an edge portion of the dressing, the reinforcement element provides a "local reinforcement" or "local rigidity" in this portion and prevents the edges of the backing layer from curling and becoming wrinkled.

The dressing may e.g. be a border dressing. A border dressing is a dressing comprising an absorbent pad, and in which the backing layer (and often also the adhesive skin-contact layer) extend beyond the edges of the pad to form a "border" portion.

The border portion of such a dressing is typically very thin and flimsy and prone to curling and wrinkling. If the machine-readable code is arranged in the border portion, the reinforcement element may locally enhance the rigidity in the border such that the area where the machine-readable code is arranged is protected from becoming wrinkled. In doing so, the readability of the machine-readable code is significantly improved.

The invention is by no means limited to the arrangement of the machine-readable code in an edge portion, e.g. a border portion, but the advantages associated with an improved readability are equally conceivable if the machine-readable code is arranged centrally on the dressing. Such an arrangement may be beneficial since irregularities in the backing layer (formed during e.g. wound exudate absorption) may generate unsuccessful readings by an electronic device.

The printed machine-readable code is arranged below the top side of the backing layer. Accordingly, the machine-readable code is prevented from becoming chafed and damaged during use, i.e. wear and tear, of the dressing.

Since the backing layer is transparent or translucent, the machine-readable code is visibly observable when the dressing is worn by a patient. Accordingly, technical information about the dressing or the manufacturing of the dressing, as well as information about the use of the dressing may be comprised in the machine-readable code, and is accessible to the patient, caregiver or medical personnel upon scanning of the machine-readable code.

The reinforcement element may be formed by any material rendering the area where the machine-readable code is arranged more rigid. The reinforcement element is arranged to directly underlie and contact the entire machine-readable code.

The reinforcement element may also be beneficial to prevent potential inks or dies from interfering with the wound site. When printed markings are arranged in the interior parts of the dressing, there is a risk that the ink or die may interfere with the wound, which is undesired. The various layers of the medical dressing are in fluid communication with the wound site. The provision of e.g. a colored ink or dye in this region may thus be associated with risks of ink or dye elution to the wound site.

However, the reinforcement element underlying the printed machine-readable code may prevent or at least significantly reduce such ink elution to the wound site.

The reinforcement element may have a surface area corresponding to the surface area of the machine-readable code, or wherein the surface area of the reinforcement element may be from 3 to 50 %, preferably from 5 to 30% larger than the surface area of the machine-readable code.

If the surface area of the reinforcement element is too large, the liquid handling of the dressing may be impaired, which could happen when the reinforcement element is arranged above the wound pad. If a too large proportion of the pad is "covered" by the reinforcement element, the transport of wound exudate through the dressing (from the wound site towards the backing layer) may be impaired. Furthermore, the flexibility of the dressing (and thereby also the stay-on ability) may be impaired.

For example, the reinforcement element may have a surface area in the range of from 80 to 500 mm², preferably from 100 to 300 mm².

Accordingly, a machine-readable code overlying the reinforcement element (or printed thereon) can thus be successfully read, i.e. scanned. The size of the machine-readable code must typically be at least 80 mm² to enable proper scanning.

In exemplary embodiments, the reinforcement element may be a label comprising a polymeric film or a nonwoven material.

The label provides a smooth and rigid surface and facilitates proper scanning of the machine-readable code throughout the use of the dressing. The label counteracts potential irregularities formed on the top side of the backing layer and secures that such irregularities are avoided or at least significantly reduced in the area where the label is arranged.

Furthermore, the label prevents curling or wrinkling of the edges of the backing layer, when the label is e.g. arranged in an edge portion, e.g. a border portion of a dressing.

In exemplary embodiments, the machine-readable code may be printed on the label.

This may be beneficial from a manufacturing point of view. It is, however, also conceivable that the machine-readable code may be printed on the bottom side of the backing layer and that the label is arranged underneath the printed machine-readable code. The benefits of providing a smooth surface and local reinforcement are equally applicable with such an arrangement.

Typically, the thickness of the label is higher than the thickness of the backing layer.

As mentioned hereinbefore, the backing layer is typically a very thin film. The thickness of the label may be at least 10% thicker, e.g. at least 30% thicker than the backing layer. For example, the thickness of the label may be from 10 to 100% thicker than the backing layer.

In exemplary embodiments, the label may have a thickness in the range of from 30 to 300 µm preferably from 50 to 200 µm.

If the label is too thin, it may not yield sufficient "protection" or contribute to the beneficial effect of improving scanning of the machine-readable code. In contrast, if the label is too thick, the difference in the thickness in the plane of the dressing may become too large.

If the interior of the dressing becomes too "uneven" or "irregular", the backing layer, when stretched during use, may create tension at the edges of the label, which may lead to detachment of the dressing from the skin.

The adhesive skin-contact layer has a top side facing the backing layer and an opposing, skin-contact side. The label may be adhesively attached to the bottom side of the backing layer or to the top side of the adhesive skin-contact layer.

The adhesive attachments secures that the label stays in place inside the dressing. For example, the bottom side of the backing layer may comprise an adhesive coating. The label, as well as the top side of the adhesive skin-contact layer and the absorbent pad (if present) may thus be adhesively attached to the bottom side of the backing layer. It is also conceivable that the label comprises an adhesive coating. Such an adhesive coating may be arranged on a top surface or a bottom surface of the label.

In alternative embodiments, the reinforcement element may be a coating having a thickness of from 30 to 300 µm, preferably from 50 to 200 µm.

The coating is typically a "locally arranged" coating configured to underlie the entire machine-readable code.

As mentioned hereinbefore, the adhesive skin-contact layer has a top side facing the backing layer and an opposing, skin-contact side. The coating may e.g. be provided on a portion of the top side of the adhesive skin-contact layer. In embodiments where the medical dressing comprises an absorbent pad, the coating may be provided on a portion of the absorbent pad.

Any coating that may locally enhance the rigidity of the dressing is conceivable.

For example, the coating may comprise at least one thermoplastic or UV curable polymer.

In exemplary embodiments, the machine-readable code may be printed on the bottom side of the backing layer.

This is particularly beneficial in embodiments where the reinforcement element is a coating. The coating is arranged to directly underlie machine-readable-code. However, as mentioned hereinbefore, it is also conceivable that the machine-readable code is printed on the bottom side of the backing layer in embodiments where the reinforcement element is a label.

As mentioned hereinbefore, printing on the bottom side of the backing layer secures that the machine-readable code is prevented from becoming chafed and damaged during use, which is typically the case with machine-readable codes provided on the top side of the dressing.

In exemplary embodiments, the medical dressing further comprises an absorbent pad arranged between the backing layer and the adhesive skin-contact layer, wherein the absorbent pad is defined by peripheral edges; the backing layer and the adhesive skin-contact layer being arranged to extend beyond the peripheral edges to form a border portion surrounding the absorbent pad, wherein the machine-readable code is arranged above the absorbent pad or above the adhesive skin-contact layer in the area forming the border portion.

The medical dressing may be a so called "border dressing". As outlined hereinbefore, the border portion has a tendency to curl or wrinkle during use. This may lead to partial detachment of the border portion from the patient's skin. If the machine-readable code is arranged in the border portion, the reinforcement element counteracts the dressing's tendency to curl and/or wrinkle in the area of the reinforcement element.

Likewise, if the machine-readable code is arranged above the absorbent pad, the absorption of exudate may cause irregularities in the backing layer. The reinforcement element counteracts the formation of such irregularities and improves the readability of the machine-readable code.

In exemplary embodiments, the adhesive skin-contact layer may comprise a plurality of perforations in the area underlying the absorbent pad but is void of perforations in the area forming the border portion.

The perforations underlying the absorbent pad secure an efficient liquid acquisition, and liquid handling in the area of the dressing where this is desirable, i.e. in the absorbent pad.

The lack of perforations in the border portion enhances the adherence of the dressing to the skin such that the dressing may stay on the skin for prolonged periods. Furthermore, a non-perforated border portion may contribute to reduced curling in the border portion.

In exemplary embodiments, the machine-readable code may be a QR code, a bar code, a data matrix code or a shot code.

The machine-readable code may encode usage-specific and/or technical information connected to the medical dressing and/or patient-specific information.

For example, the machine-readable code may encode a link to instructions on how the dressing should be used, applied etc. Alternatively, the machine-readable code may encode information about the batch number, production lot number, and/or country indication. Such information may be beneficial for regulatory purposes, anti-counterfeiting and potential product recall purposes and enables the authentication of the product. Accordingly, each dressing may be tracked and traced, e.g. back to the earliest steps of manufacturing.

The country indication may be useful for preventing illegal imports, i.e. branded products being imported into a market and sold there without the trademark owner's consent in that market or country.

It is also conceivable that the machine-readable code encodes patient-specific information, e.g. a link to a patient's database or journal, which may contain health care data of the patient.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF DRAWINGS

The various aspects of the present disclosure, including its particular features and advantages, will be readily understood from the following detailed description and the accompanying drawings, in which:
Figure 1 schematically illustrates a medical dressing according to an exemplary embodiment of the present disclosure. In figure 1, a portion of the backing layer has been cut out to illustrate the provision of a reinforcement element in the form of a label in this area.
Figure 2a is the medical dressing of figure 1, as seen from the top side of the backing layer.
Figure 2b is the medical dressing of figure 1 as seen from the skin-contact side of the adhesive skin-contact layer.
Figure 2c schematically illustrates a medical dressing according to an exemplary embodiment of the present disclosure, wherein the machine-readable code and the reinforcement element are arranged above the absorbent pad.

### DETAILED DESCRIPTION

The present disclosure will now be described more fully hereinafter with reference to the accompanying drawings, in which currently preferred embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person. Like references refer to like elements throughout.

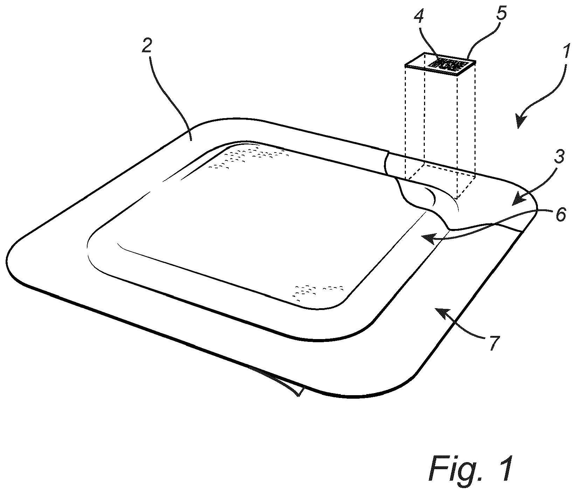

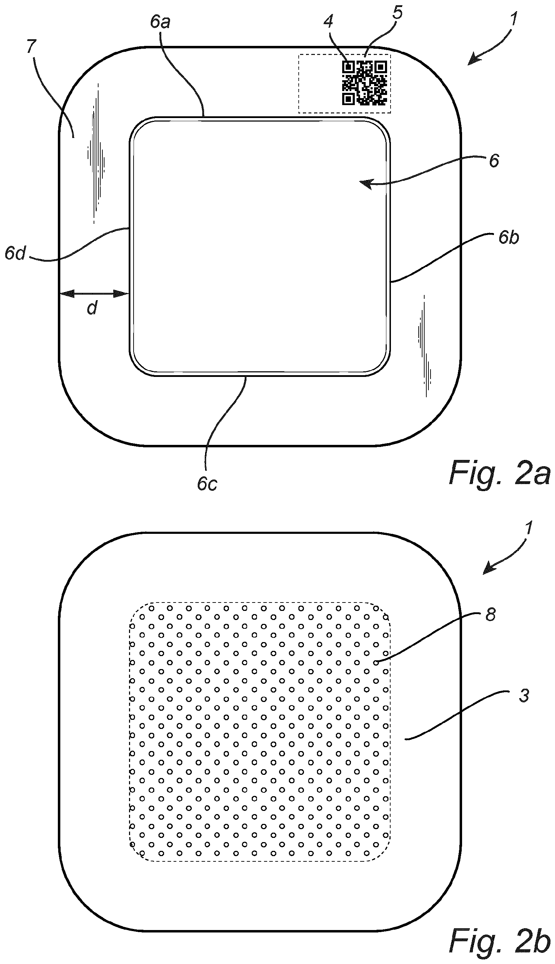

An exemplary medical dressing is schematically illustrated in figure 1 and figures 2a-c. The medical dressing 1 comprises at least a backing layer 2 and an adhesive skin-contact layer 3; the backing layer 2 being transparent or translucent and having a top side and a bottom side, wherein the medical dressing comprises a machine-readable code 4 arranged between the top side of the backing layer 2 and the adhesive skin-contact layer 3, wherein the medical dressing 1 further comprises a reinforcement element 5 in direct contact with and below the machine-readable code 4.

As used herein, the term "reinforcement element" means a material section, such as a label or a coating, which provides additional structural support in an area of the dressing, i.e. in the area underlying the machine-readable code. The primary function of the reinforcement element is to maintain the flatness and integrity of the dressing surface in the area underlying the machine-readable code. The reinforcement element counteracts the mechanisms which may compromise the readability of the machine-readable code during use. Such mechanisms include wrinkles, curling of dressing edges, and irregularities formed due to absorption of wound exudate.

The reinforcement element 5 may have a surface area in the range of from 80 to 500 mm², preferably from 100 to 300 mm².

The surface area of the reinforcement element may vary depending on the size of the dressing and on the size of the machine-readable code. Medical dressings come in a variety of sizes and a variety of shapes. The size of the reinforcement element may consequently vary.

However, in order to secure readability of the machine-readable code when scanned by an electronic device, the surface area of the machine-readable code is typically at least 80 mm². To secure coverage of the machine-readable code, the surface area of the reinforcement element 5 is thus at least 80 mm².

The term "machine-readable code" is a code configured to store information in a format that machines, e.g. electronic devices, can quickly and accurately decode. Examples of machine-readable codes include QR codes, barcodes, data matrix codes, and shot codes.

The information encoded by the machine-readable code may be usage-specific and/or technical information connected to the medical dressing, or patient-specific information.

The machine-readable code may e.g. encode a link to technical and/or usage-specific information connected to the medical dressing and/or a link to patient-specific information.

As used herein the term "usage-specific information" means data, instructions, guidelines or specifications that pertain to the proper use, functionality and handling of the medical dressing. The information is intended to aid healthcare providers and patients in an effective and safe use of the dressing. Usage-specific information may include instructions for application and removal of the dressing, indications for use, precautions, guidance on storage conditions, handling procedures and disposal methods.

The term "technical information" may include details about the composition, materials, dimensions, and manufacturing process of the medical dressing. This may also include data sheets, technical specifications, performance characteristics and compliance with regulatory standards. For example, the technical information may include information about the batch number, production lot number, and/or country indication, which may be useful for e.g. product recall, regulatory and/or counterfeiting purposes.

As used herein, the term "patient-specific information" means information or data that may be unique to an individual patient. Such information may include personal medical data, such as information about the patient's name, age, medical history, allergies, specific wound characteristics (e.g. wound type, size, location, healing stage etc.). Patient-specific information may also include treatment plans, e.g. dressing change schedules, medication or topical treatments applied in conjunction with the dressing, and potential progress notes (e.g. observations of wound healing, adverse reactions etc.) from medical personnel. Furthermore, such information may also include a link to the patient's journal or health records.

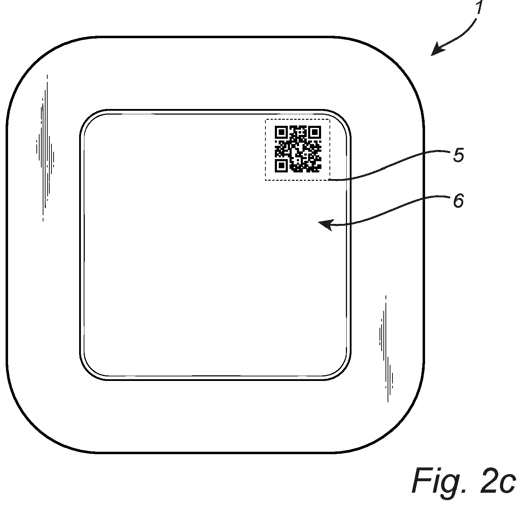

The machine-readable code 4 may be square-shaped, as illustrated in figures 1, 2a, and 2c. It is also conceivable that the machine-readable code has a rectangular or a curvilinear shape, e.g. a circular or an oval shape.

The machine-readable code 4 may have a surface area in the range of from 80 to 500 mm², preferably from 100 to 300 mm².

The backing layer 2 is translucent or transparent. Hence, light is allowed to pass through the backing layer such that the printed machine-readable code 4 can be visibly observed and scanned during use.

The printed machine-readable code is arranged below the top side of the backing layer 2. Accordingly, the risk of chafing the machine-readable code during use is eliminated. The readability of the machine-readable code is thereby protected.

In e.g. figure 1, the reinforcement element is a label.

The label may comprise a polymeric film or a nonwoven.

The polymeric film may comprise polyethylene, polyamide, polyester, and/or polyurethane.

A polymeric film may be beneficial in embodiments wherein the dressing comprises an absorbent pad (as illustrated in figures 1 and 2a-c), and the label is arranged above the absorbent pad (see figure 2c). A polymeric film is generally non-absorbing. In this regard, wound exudate is not absorbed by the label, and the protective function of the label is thus not impaired when exudate is transferred through the absorbent pad.

Furthermore, a polymeric film may prevent or at least significantly reduce potential ink elution to the wound site.

A nonwoven label may be used in embodiments where the label is arranged in an area of the dressing where less wound exudate is present or on low-exuding wounds.

For example, a nonwoven label may be arranged in an edge portion of the dressing.

As illustrated in the "border dressing" in figure 1, the nonwoven label may be arranged in the border portion 7 of the dressing. The border portion is typically arranged remote from the wound site. If a nonwoven label is arranged in the area overlying the absorbent pad 6, exudate may be absorbed by the nonwoven such that the readability of the machine-readable code becomes impaired.

The thickness of the label may be higher than the thickness of the backing layer. For example, the thickness of the label may be at least 15 % higher, e.g. at least 40%, such as at least 100% higher than the thickness of the backing layer.

The thickness of the label may be in the range of from 30 to 300 µm, preferably from 50 to 200 µm.

The thickness is measured in dry conditions.

If the thickness of the label is too high, this may negatively affect the stay-on ability of the dressing onto the skin. If the interior of the dressing becomes too uneven or irregular, the backing layer, when stretched during use, may create tension at the edges of the label, which may lead to detachment of the dressing from the skin.

The label is typically adhesively attached to the backing layer, the absorbent pad, if present, or the adhesive skin-contact layer.

In exemplary embodiments, an adhesive coating may be arranged on the bottom side of the backing layer. The adhesive coating secures attachment of the backing layer to the adhesive skin contact layer, and to the absorbent pad, if present. The adhesive coating provided on the bottom side of the backing layer may be used to adhesively attach the label.

The adhesive skin-contact layer 3 has a top side facing the backing layer and a second, skin-contact side. The top side of the adhesive skin-contact layer is illustrated in figure 1. The skin-contact side (bottom side) of the adhesive skin-contact layer is illustrated in figure 2b.

The label has a top side facing the backing layer 2 and a bottom side facing the adhesive skin-contact layer 3 (and the absorbent pad 6, if present). The machine-readable code is provided on the top side of the label.

The adhesive attachment may also be provided by an adhesive coating arranged on the bottom side of the label. This way, the label may be adhesively attached to the adhesive skin-contact layer or to the absorbent pad (if present).

In alternative embodiments, the reinforcement element is a coating having a thickness of from 30 to 300 µm, preferably from 50 to 200 µm.

The present disclosure is not limited to a specific coating material as long as the coating can reinforce, i.e. render the area underlying the printed machine-readable code more rigid and smooth.

For example, the coating may comprise at least one thermoplastic or UV curable polymer.

For example, the thermoplastic polymer may be selected from the group consisting of thermoplastic polyurethane, polyethylene, and/or polypropylene.

The UV curable polymer may be selected from the group consisting of acrylates, epoxy resins, and silicones.

The coating may be adhesive or non-adhesive. Preferably, the coating is adhesive.

The dimensions of the coating, i.e. the thickness and surface area typically corresponds to the dimensions of the label as mentioned hereinbefore.

In figures 2a and 2c, the reinforcement element 5 is denoted with dotted lines. The dotted lines may indicate the surface area of the coating.

In figure 1, the machine-readable code 4 is printed on the label. This may be beneficial from a manufacturing point of view.

Alternatively, the machine-readable code may be printed on the bottom side of the backing layer.

The printed machine-readable code may be formed by a colored ink (regardless of being printed on the label or on the bottom side of the label).

Any colored ink may be used. The ink may be water based or solvent based, i.e. the ink may comprise an organic solvent, such as an alcohol, ester etc. that can dissolve the pigment, resin and potentially other additives. For example, a flexographic ink may be used. The machine-readable code 4 may be printed by means of any conventional printing technique known in the art, including, but not limited to a gravure printing, a flexographic printing, an offset printing, thermal transfer printing, an ink jet printing and the like.

It is also conceivable that the machine-readable code is printed by UV laser printing. The printed machine-readable code may thus be formed by one or a plurality of UV laser beams. The wavelength of the UV laser is typically in the range of from 320 to 380 nm, preferably at or about 355 nm.

Printing with UV laser is beneficial since printing may be accomplished through the top side of the backing layer, i.e. after the dressing has been assembled.

In such embodiments, the reinforcement element is typically a label comprising a polymeric film, wherein the polymeric film comprises a UV absorbing polymer.

The label (arranged below the top side of the dressing) will absorb the UV laser light such that the printed machine-readable code is formed on the label. The UV-absorbing polymeric material undergoes a photolytic reaction such that the polymer bonds break, and a visible printed marking (machine-readable code) is provided on the label. In such embodiments, the backing layer is typically transparent and non-UV absorbing. Accordingly, no print will be formed on the top side of the label.

In embodiments where the reinforcement element is a label, the label may be transparent or white. Accordingly, the contrast between the printed machine-readable code and the label is improved. A white label may be preferred to improve the visibility of the machine-readable code when the dressing has become moistened with wound exudate. A white label is also preferably used if the absorbent pad is gray (which may be the case for e.g. antimicrobial dressings).

In embodiments where the reinforcement element is a coating, the machine-readable code is typically printed on the bottom side of the backing layer. The coating may be arranged on the adhesive skin contact layer or on the absorbent pad in an area directly underlying the printed machine-readable code.

As illustrated in figures 1 and 2a-c, the medical dressing may further comprise an absorbent pad 6 arranged between the backing layer 2 and the adhesive skin-contact layer 3, wherein the absorbent pad 6 is defined by peripheral edges 6a-d; the backing layer 2 and the adhesive skin-contact layer 3 being arranged to extend beyond the peripheral edges 6a-d to form a border portion 7 surrounding the absorbent pad 6, wherein the machine-readable code 4 is arranged above the absorbent pad 6 (see figure 2c) or above the adhesive skin-contact layer 3 in the area forming the border portion 7 (see figure 2a).

In the context of the present disclosure, the "absorbent pad" may comprise one or a plurality of pad-forming layers.

The absorbent pad may e.g. comprise an absorbent polyurethane foam layer.

The absorbent pad may further comprise a superabsorbent layer, i.e. a layer comprising a superabsorbent material. The superabsorbent material may be superabsorbent polymer (SAP) particles or superabsorbent fibres (SAF). The superabsorbent material is capable of handling large amounts of wound exudate.

The absorbent pad may also comprise a liquid distribution layer. The liquid distribution layer may comprise any material having the ability to distribute the exudate in an efficient manner. For example, the liquid distribution layer may comprise a nonwoven material.

A layered pad construction prevents accumulation of body liquids close to the skin and improves the overall liquid handling of the medical dressing.

As best illustrated in figure 2a, the absorbent pad 6 is defined by peripheral edges 6a-d. The peripheral edges of absorbent pad 6 may be straight or curved.

The backing layer 2 is attached to the adhesive skin-contact layer 3 in the area of the border portion 5. For example, the backing layer 2 may be attached to the adhesive skin-contact layer 3 by an adhesive, e.g. a polyacrylate adhesive. Heat lamination of the backing layer 2 and adhesive skin-contact layer is also conceivable.

The backing layer 2 and the adhesive skin-contact layer 3 are co-extensive, i.e. they have the same length and width. This is the case also in embodiments where the medical dressing is void of an absorbent pad.

The size of the absorbent pad 8 is smaller than the size of the backing layer 2 and the adhesive skin-contact layer 3.

The border portion 7 surrounds the absorbent pad. In other words, the border portion extends a distance, d, beyond each peripheral edge of the absorbent pad 4 (see figure 2a).

The distance, d, may be the same or it may differ. For example, if the dressing has a more "complex" shape, as is the case with e.g. heel or sacral dressings, some parts of the medical dressing may have a larger border portion. Accordingly, the distance, d, may vary along the peripheral edges of the medical dressing. Alternatively, the distance, d, is the same around the whole periphery of the absorbent pad.

In order to secure a firm attachment to the skin and to secure sufficient space for the machine-readable code and the reinforcement element, the border portion may have a width of at least 10 mm, e.g. from 10 to 50 mm.

A smaller sized dressing may have a smaller border portion than a larger sized dressing.

As best illustrated in figure 2b, the adhesive skin-contact layer 3 may comprise a plurality of perforations 8 in the area underlying the absorbent pad 6 but is void of perforations in the area forming the border portion 7.

The area of the adhesive-skin contact layer 3 forming the border portion 7 (see figure 2b) is preferably non-perforated. Hence, the entire border portion 5 is void of perforations. This is beneficial to improve the adhesion to the skin in the border of the dressing such that the stay-on ability of the dressing is improved.

The perforations 8 in the adhesive skin-contact layer 3 arranged underneath the absorbent pad 4 improve the absorption of wound exudate and liquid handling of the dressing and are therefore arranged in the area where absorption takes place.

The perforations may be of various shapes and sizes. Preferably, the perforations are arranged in a predetermined, regular pattern. The perforations may have a diameter of from 0.5 mm to 10 mm, e.g. from 1 to 5 mm, preferably from 1 to 2 mm.

In figures 1, and 2a-b, the dressing is substantially square shaped. However, the medical dressing of the present disclosure is not limited to a particular shape, but any shape is conceivable, such as rectangular, oval, round etc. It is also conceivable that the medical dressing has a shape adapted to fit with the sacrum or heel of a wearer.

The backing layer 2 and the adhesive skin-contact layer 3 are co-extensive, i.e. they have the same length and width.

The maximum width, i.e. maximum lateral (x) extension of the dressing may be from 35 to 250 mm.

The maximum length, i.e. maximum longitudinal (y) extension of the dressing may be from 50 to 450 mm.

The medical dressing typically further comprises a release liner detachably attached to the adhesive skin-contact layer. The release liner may comprise one or a plurality of release liner portion and is configured to cover the entire surface of the adhesive skin-contact layer 3.

In the context of the present disclosure, the "backing layer" is the top layer of the dressing, i.e. the outermost layer of the dressing. The backing layer is a continuous layer. In other words, the backing layer is void of perforations and incisions. The backing layer protects the layers of the dressing from potential contaminants.

The backing layer is desirably a thin and pliable layer such that it can stretch and conform to the movement of a wearer. The backing layer may be a polymeric film. Suitably, the backing layer comprises a polyurethane film. The backing layer may also be a laminate of a nonwoven layer and at least one polyurethane film.

The thickness of the backing layer may be in the range of from 15 to 50 µm, preferably from 20 to 40 µm.

The "adhesive skin-contact layer" of the medical dressing is configured to detachably adhere the dressing to a dermal surface. In other words, the adhesive skin-contact layer is configured to contact the skin or the wound of a wearer. This layer may also be referred to as a "wound contact layer".

The adhesive skin-contact layer 3 has a top side facing the backing layer 2, and an opposing bottom side (skin-contact side). In figure 1, the top side of the adhesive skin-contact layer is visible in the cut-out portion of the medical

The adhesive-skin contact layer 3 may comprise a polymeric film and an adhesive silicone gel; the silicone gel being arranged to contact the skin of a wearer during use.

An adhesive skin-contact layer comprising a silicone gel is skin-friendly and easy to remove without causing trauma. It is sufficiently adherent to skin such that the dressing stays in place, but is configured to maintain its adherence with repeated removal and re-application.

Examples of suitable silicone gels for use in the adhesive skin-contact layer include the two component RTV systems, such as Q72218 (Dow Corning), and SilGel 612 (Wacker Chemie AG), as well as NuSil silicone elastomers. In embodiments of the invention the adhesive may comprise a soft silicone gel having a softness (penetration) of from 8 to 22 mm, e.g. from 12 to 17 mm, as measured by a method based on ASTM D 937 and DIN 51580, the method being described in European Patent Application No 14194054.4. The thickness of the adhesive skin-contact layer is typically at least 20 µm. The thickness of the adhesive skin-contact layer may be from 50 to 200 µm.

The polymeric film may be a breathable film and may comprise e.g. polyethylene, polyamide, polyester or polyurethane. Preferably, the polymer-based film comprises polyurethane. The thickness of the polymeric film may be from 15 to 100 µm, e.g. from 20 to 80 µm, preferably from 20 to 60 µm.

The adhesive skin-contact layer 3 may be translucent or transparent to improve the visibility of the printed machine-readable code 4.

Hence, the printed machine-readable code 4 may be visibly observable when viewed from the bottom-side of medical dressing, i.e. from the bottom side of the adhesive skin contact layer 3. In figure 2b, the medical dressing is seen from the bottom-side of the medical dressing. For ease of illustration, the machine-readable code is not shown in this view.

Terms, definitions and embodiments of all aspects of the present disclosure apply mutatis mutandis to the other aspects of the present disclosure.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A medical dressing (1) comprising at least a backing layer (2) and an adhesive skin-contact layer (3); said backing layer (2) being transparent or translucent and having a top side and a bottom side, wherein said medical dressing comprises a machine-readable code (4) arranged between said top side of said backing layer (2) and said adhesive skin-contact layer (3), **characterized in that** said medical dressing (1) further comprises a reinforcement element (5) in direct contact with and below said machine-readable code (4).

2. The medical dressing (1) according to claim 1, wherein said reinforcement element (5) is a label or a coating.

3. The medical dressing according to claim 1 or claim 2, wherein said reinforcement element (5) has a surface area corresponding to the surface area of said machine-readable code (4), or wherein the surface area of said reinforcement element (5) is from 3 to 50 %, preferably from 5 to 30% larger than the surface area of said machine-readable code (4).

4. The medical dressing according to any one of the preceding claims, wherein said reinforcement element (5) has a surface area in the range of from 80 to 500 mm², preferably from 100 to 300 mm².

5. The medical dressing (1) according to any one of the preceding claims, wherein said reinforcement element (5) is a label comprising a polymeric film or a nonwoven material.

6. The medical dressing (1) according to claim 5, wherein said machine-readable code (4) is printed on said label.

7. The medical dressing according to claim 5 or claim 6, wherein the thickness of said label is higher than the thickness of said backing layer (2).

8. The medical dressing (1) according to any one of claims 5-7, wherein said label has a thickness in the range of from 30 to 300 µm, preferably from 50 to 200 µm.

9. The medical dressing (1) according to any one of claims 5-8, wherein said adhesive skin-contact layer (3) has a top side facing said backing layer (2) and an opposing, skin-contact side, wherein said label is adhesively attached to said bottom side of said backing layer (2) or to said top side of said adhesive skin-contact layer (3).

10. The medical dressing (1) according to any one of claims 1-3, wherein said reinforcement element (5) is a coating having a thickness of from 30 to 300 µm, preferably from 50 to 200 µm.

11. The medical dressing (1) according to claim 10, wherein said coating comprises a thermoplastic or a UV curable polymer.

12. The medical dressing according to any one of 1-5 or claims 7-11, wherein said machine-readable code (4) is printed on said bottom side of said backing layer (2).

13. The medical dressing according to any one of the preceding claims, further comprising an absorbent pad (6) arranged between said backing layer (2) and said adhesive skin-contact layer (3), wherein said absorbent pad (6) is defined by peripheral edges (6a-d); said backing layer (2) and said adhesive skin-contact layer (3) being arranged to extend beyond said peripheral edges (6a-d) to form a border portion (7) surrounding said absorbent pad (6), wherein said machine-readable code (4) is arranged above said absorbent pad (6) or above said adhesive skin-contact layer (3) in the area forming said border portion (7).

14. The medical dressing according to claim 13, wherein said adhesive skin-contact layer (3) comprises a plurality of perforations (8) in the area underlying said absorbent pad (6) but is void of perforations in the area forming said border portion (7).

15. The medical dressing (1) according to any one of the preceding claims, wherein said machine-readable code is a QR code, a bar code, a data matrix code or a shot code.

16. The medical dressing (1) according to any one of the preceding claims, wherein said machine-readable code encodes usage-specific and/or technical information connected to the medical dressing and/or patient-specific information.
